# EUROPEAN PATENT SPECIFICATION

(11) **EP 4 639 151 B1**
(45) Date of publication and mention of the grant of the patent: **11.11.2026**
(21) Application number: 23836399.8
(22) Date of filing: 19.12.2023
(51) Int. Cl.: G01N 21/84

(54) **ANALYTICAL METHOD OF DETERMINING AT LEAST ONE PROPERTY OF A SAMPLE OF A BODILY FLUID**
ANALYTISCHES VERFAHREN ZUR BESTIMMUNG MINDESTENS EINER EIGENSCHAFT EINER PROBE EINER KÖRPERFLÜSSIGKEIT
PROCÉDÉ ANALYTIQUE POUR DÉTERMINER AU MOINS UNE PROPRIÉTÉ D'UN ÉCHANTILLON D'UN FLUIDE CORPOREL

(30) Priority: 21.12.2022 EP 22215279
(43) Date of publication of application: 29.10.2025
(73) Proprietor: F. Hoffmann-La Roche AG, 4070 Basel (CH); Roche Diabetes Care GmbH, 68305 Mannheim (DE)
(72) Inventor: BERG, Max, 68305 Mannheim (DE); HAILER, Fredrik, 68305 Mannheim (DE); LIMBURG, Bernhard, 68305 Mannheim (DE)
(74) Representative: Riwotzki, Karsten
(86) International application number: PCT/EP2023/086548
(87) International publication number: WO 2024/133203

(56) References cited:
- US-A1- 2019 302 008
- US-A1- 2020 126 226
- US-A1- 2020 225 166
- US-A1- 2021 172 945
- US-A1- 2022 270 720
- US-B1- 10 928 325

## Description

### Technical Field

The present invention refers to an analytical method, specifically an *in vitro* analytical method, of determining at least one property of at least one sample of a bodily fluid, to a method of operating a first mobile device and to a method of operating a second mobile device. The invention further relates to a first mobile device, a second mobile device, an analytical system comprising the first mobile device and the second mobile device, and to computer programs and computer-readable storage media for performing the methods. The methods and devices may specifically be used in medical diagnostics in order to quantitatively or qualitatively detect one or more properties of a sample of a bodily fluid. As an example, the methods and devices may be used for detecting the presence of the SARS-CoV-2 coronavirus in a sample of a bodily fluid. Other fields of application of the present invention, however, are also feasible.

### Background art

In the field of medical diagnostics, in many cases, samples of a bodily fluid, such as saliva, blood, interstitial fluid, urine or other types of bodily fluids, have to be analyzed, for example in order to detect a presence and/or a concentration of an analyte in the sample of the bodily fluid. Examples of analytes to be detected are viruses, such as the SARS-CoV-2 coronavirus. However, the present invention may also be used for other types of analytes, such as glucose, triglycerides, lactate, cholesterol or other types of analytes typically present in these body fluids. Without narrowing the scope, the invention specifically may be described with respect to the detection of the SARS-CoV-2 coronavirus.

Generally, devices and methods known to the skilled person make use of test elements comprising one or more test chemicals, which, in presence of the analyte to be detected, are capable of performing one or more detectable detection reactions, such as optically detectable detection reactions. As an example, WO2022/049440 A1 discloses an apparatus including a mobile computing device physically coupled to a lightbox. The apparatus includes camera hardware configured to capture image data associated with an output signal area of a biological chromatographic test strip inserted into a receiving slot of the lightbox. The apparatus further includes processing circuitry in communication with the camera hardware, the processing circuitry being configured to determine, based on the image data captured by the camera hardware, a concentration of a target analyte in a test sample submitted via the biological chromatographic test strip. The apparatus further includes an interface in communication with the processing circuitry, the interface being configured to output data indicative of the concentration of the target analyte determined by the processing circuitry.

US 2021/0241456 A1 and the corresponding US 2020/126226 A1 describe systems and methods for analyzing visible chemical reactions. In one implementation, the method may include receiving from an image sensor associated with a mobile communications device an image of a reagent pad in proximity to a colorized surface having at least one pair of colored reference elements. The method includes identifying in the image the reagent pad, a first colored reference element, and a second colored reference element. Thereafter, the method includes using the first colored reference element and the second colored reference element to determine an extent of a chemical reaction on the reagent pad irrespective of local illumination conditions. Then the method includes causing the mobile communications device to provide data based on the extent of the chemical reaction.

US 11,060,968 B2 discloses a method comprising receiving a target sample to be chemically analyzed at a microfluidic device which is part of an analysis card. The microfluidic device includes at least one input layer configured to receive the target sample, at least one intermediary layer, and at least one readout layer configured to present one or more color attributes at one or more readout regions in response to one or more colorimetric reactions to the target sample. An image of the readout layer of the microfluidic device is obtained. A calibration file that corresponds to the analysis card is obtained. The calibration file includes a calibrated model of at least one color attribute based on data from at least one known chemical attribute of at least one standard sample processed by a calibration card. A data processing function is executed on the obtained image by comparing the one or more color attributes at the one or more readout regions to the calibrated model of the at least one color attribute to determine at least one chemical attribute of the target sample.

WO 2020/123858 A1 describes techniques and apparatus for capturing an image of a person's retina fundus, identifying the person, accessing various electronic records (including health records) or accounts or devices associated with the person, determining the person's predisposition to certain diseases, and/or diagnosing health issues of the person. Some embodiments provide imaging apparatus having one or more imaging devices for capturing one or more images of a person's eye(s). Imaging apparatus described herein may include electronics for analyzing and/or exchanging captured image and/or health data with other devices. In accordance with various embodiments, imaging apparatus described herein may be alternatively or additionally configured for biometric identification and/or health status determination techniques.

WO 2021/138477 A1 describes methodologies and technologies for determining changes in one or more skin conditions of a user over time. Any changes in skin conditions over time may be used as a diagnosis and/or treatment aid for a physician. Any changes in skin conditions over time may be also used in a computer implemented method that provides diagnosis and/or treatment recommendations. In some examples, improved image quality is attained via calibration techniques and methodologies. In these examples, improved image accuracy and quality is provided by addressing issues relating to unpredictable and inconsistent lighting conditions, among others. In an example, the system includes a mobile computing device and an object with known lighting and/or color attributes (e.g., a reference). Such an object acts as a calibration device for images to be captured by the mobile computing device.

US 2021/0073681 A1 discloses methods, apparatuses, systems, and computer-readable media for identifying and executing one or more interactive condition evaluation tests and collecting and analyzing user behavior data to generate an output. In some examples, user information may be received and one or more interactive condition evaluation tests may be identified. An instruction may be transmitted to a computing device of a user and executed on the computing device to enable functionality of one or more sensors that may be used in the identified tests. Upon initiating a test, data may be collected from the one or more sensors. The collected sensor data may be transmitted to the system and processed using one or more machine learning datasets. Additionally, user behavior data may be collected and processed using one or more machine learning datasets. The sensor data, the user behavior data, and other data may be used together to generate an output.

A wide variety of tests systems for testing samples for the presence or concentration of viruses are generally known. For point-of-care (POC) testing or for other situations where a quick result is desirable, a variety of lateral flow (LF) tests detecting viral antigens are commercially available. For example, in the case of SARS-CoV-2, LF test detecting the nucle-ocapsid antigen are widely used, but also the spike antigen may be detected. However, also tests detecting viral nucleic acids have been proposed as lateral flow tests, cf. e.g. WO 2021/228839 and references cited therein, which are also referred to as "SHERLOCK" tests.

Further, instead of blood tests, saliva was proposed as a suitable sample material for SARS-CoV-2 detection (Wyllie et al. (2020), medRxiv 2020.04.16.20067835; doi.org/10.1101/2020.04.16.20067835). B. Ince and M. K. Sezgintürk provide in "Lateral flow assays for viruses diagnosis: Up-to-date technology and future prospects", 2022, TrAC Trends in Analytical Chemistry, Volume 157, 116725, an overview of current problems and accessible solutions in detecting infectious agents and diseases by lateral flow assay (LFA), focusing on increasing sensitivity with various detection methods. Further, the sensitivity of SARS-CoV-2 antigen rapid diagnostic tests (Ag RDT) was evaluated in "Comparative sensitivity evaluation for 122 CE-marked rapid diagnostic tests for SARS-CoV-2 antigen, Germany, September 2020 to April 2021", H. Scheiblauer et al., Eurosurveillance, 26, 2100441 (2021).

US 2020/0225166 A1 discloses a reagent test paddle including a contamination detection medium, a reference color bar, at least one chemical test medium, and a unique identifier. The contamination detection medium includes a reagent that changes color in the presence or when exposed to a hostile or inhospitable environment. Each chemical test medium includes a reagent that is responsive to a respective analyte in a biological sample. The reference color bar includes reference color samples of different colors. The unique identifier, like a serial number, identifies the particular paddle and its chemical test medium so it can be uniquely and anonymously associated with a user. A method includes capturing and interpreting digital images of a biologically unexposed and subsequently exposed reagent test paddle at various delay times within an automatically calibrated environment; locating the paddle in a plurality of digital images; extracting the reference color bar and locating the contamination detection medium and chemical test medium in each digital image. Color changes of the chemical test medium and contamination medium are detected at various delay times after sample exposure. To determine validity of test results, the method further compares the detected colors of the contamination detection medium with predetermined colors expected for no contamination and contamination.

US 10,928,325 B1 describes a system that makes nutritional recommendations based on results of a home urine test. A user may apply a urine sample to a card containing multiple tests, and capture an image of the card using a phone; an analysis system executing on the phone or in the cloud may analyze the image and determine test results. The test card and analysis system may compensate for variability in lighting conditions and time of exposure to the urine sample. Based on test results, the system may recommend consumption of specific quantities of nutrients, such as vitamins, minerals, and foods. It may also recommend consumption of water or electrolytes based on measured hydration, and stress reduction techniques or sleep based on measured cortisol. Recommendations may be customized based on factors such as the user's characteristics (gender, weight, etc.), predicted absorption of nutrients from food or supplements, and the user's dietary preferences or restrictions.

Generally, when using mobile devices, such as one or more smartphones or the like, as diagnostic devices for evaluating optically detectable detection reactions using optical test elements, the full physical measurement sequence, i.e. the measurement sequence irrespective of any potential cloud supported calculations etc., may be run on the very same mobile device from start to end. For safety reasons, for example to ensure a valid, unused, functional test element and/or appropriate environmental conditions, the standard measurement procedure of many optical test elements may include a first initial scan prior to any sample application, a second scan after sample application and a defined waiting or reaction time in between the first and the second scan.

However, despite the advantages achieved by known methods and devices, several technical challenges remain. For example, in Covid-19 test centers or other point of care scenarios, the mobile device-based measurement may not be carried out by an individual patient or customer but by one or more dedicated persons using one or more mobile devices. In these scenarios, a plurality of tests may be run in parallel especially due to the typical waiting time. Thus, running the full physical measurement sequence using one and the very same mobile device would require a strict organizational assignment of tests and executing staff. This is generally error-prone and/or reduces the overall throughput of performed measurements.

### Problem to be solved

It is therefore desirable to provide methods and devices which at least partially address the above-mentioned technical challenges. Specifically, methods and devices shall be proposed which allow a user-friendly and robust analytical method of determining at least one property of at least one sample of a bodily fluid. More specifically, methods and devices shall be proposed which may allow for a mass-testing scenario, specifically in one or more test centers, which, despite of a possible high throughput of e.g. 50 or more tests per day, is safe and has a low error rate.

### Summary

This problem is addressed by an analytical method of determining at least one property of at least one sample of a bodily fluid, by a method of operating a first mobile device, by a method of operating a second mobile device, by a first mobile device and a second mobile device, by an analytical system comprising the first mobile device and the second mobile device, and by computer programs and computer-readable storage media for performing the methods with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, an analytical method, specifically an *in vitro* analytical method, of determining at least one property of at least one sample of a bodily fluid, specifically for detecting at least one analyte in the bodily fluid, is disclosed.

The term "analytical method" as used herein is a broad term and is to be given its ordinary and customary meaning to a person of ordinary skill in the art and is not to be limited to a special or customized meaning. The term specifically may refer, without limitation, to a quantitative and/or qualitative determination of the at least one property of the sample of the bodily fluid, such as at least one of a physical, a chemical and a biological property. The determination of the at least one property may specifically comprise quantitatively or qualitatively detecting at least one analyte in the sample of the bodily fluid. The result of the analytical method, as an example, may be at least one item of information on the at least one property of the sample, such as at least one item of information on the presence or absence of an analyte of interest. Alternatively or additionally, the result of the analytical method may comprise at least one item of information indicating a concentration of the at least one analyte of interest. The analyte may be or may comprise at least one arbitrary, dedicated and/or predetermined chemical or biological substance or species, such as at least one molecule or at least one chemical and/or biological compound. For example, the analyte may be or may comprise at least one specific virus and/or any parts thereof. The result of the analytical method may be or may comprise at least one item of information indicating the presence or absence of the virus or parts thereof in the sample of the bodily fluid. For example, the analyte may be a chemical compound which takes part in metabolism, such as one or more of glucose, lactate, cholesterol or triglycerides. In this example, the analytical method may be a blood glucose measurement and, thus, the result of the analytical method may for example be a blood glucose concentration. Additionally or alternatively, other types of analytes or parameters may be determined, such as a pH value or the like. As will be understood by the skilled person, a "presence" of an analyte, for example, may be a presence of said analyte in an amount above a detection limit of the test used.

The term "*in vitro*" as used herein is a broad term and is to be given its ordinary and customary meaning to a person of ordinary skill in the art and is not to be limited to a special or customized meaning. The term specifically may refer, without limitation, to a method which uses components of an organism and which is at least partially performed isolated from a usual biological surroundings of the components of the organism. For example, the *in vitro* analytical method may comprise using the sample of the bodily fluid isolated from a user or patient, specifically using the sample of the bodily fluid in at least one optical test element, as will be outlined in further detail below.

The analytical method specifically may relate to a method for detecting an RNA virus in a sample of bodily fluid. The method specifically may comprise the steps of releasing viral RNA from the sample, amplifying at least parts of the viral RNA comprised in said sample, and contacting the amplified viral RNA with at least one detecting reagent, such as at least one detecting reagent containing at least one nuclease, such as in the Sherlock tests described herein above. As an example, the analytical method may comprise binding a viral antigen to an antibody, which may comprise a detectable label, such as a gold particle, a dye or the like. As another example, the analytical method may comprise performing a lateral flow test; appropriate methods are known in the art.

The term "property" as used herein is a broad term and is to be given its ordinary and customary meaning to a person of ordinary skill in the art and is not to be limited to a special or customized meaning. The term specifically may refer, without limitation, to at least one item of information indicating a state or a characteristic of the sample of the bodily fluid. The property may be an intrinsic property of the sample and, thus, may be determined in a measurement, such as by using the analytical method. The property may specifically be an optically detectable property, such as a property of the sample of the bodily fluid causing an optical detectable detection reaction. For example, the at least one property of the sample may comprise at least one of the presence of at least one predetermined analyte in the bodily fluid and the concentration of at least one predetermined analyte in the bodily fluid. For example, the property of the sample may be selected from the group consisting of: the presence of at least one predetermined type of virus in the sample, specifically the presence of at least one predetermined type of RNA virus, more specifically the presence of the SARS-CoV-2 coronavirus and/or the presence of the Influenza virus A and/or the presence of the Influenza virus B; the concentration of at least one predetermined type of virus in the sample, specifically the concentration of at least one predetermined type of RNA virus, more specifically the concentration of the SARS-CoV-2 coronavirus and/or the concentration of the Influenza virus A and/or the concentration of the Influenza virus B; the presence of at least one predetermined type of antibody in the sample, specifically the presence of at least one predetermined type of antibody against at least one RNA virus, more specifically the presence of at least one antibody against the SARS-CoV-2 coronavirus and/or the presence of at least one antibody against the Influenza virus A and/or the presence of at least one antibody against the Influenza virus B; the concentration of at least one predetermined type of antibody in the sample, specifically the concentration of at least one predetermined type of antibody against at least one RNA virus, more specifically the concentration of at least one antibody against the SARS-CoV-2 coronavirus and/or the concentration of at least one antibody against the Influenza virus A and/or the concentration of at least one antibody against the Influenza virus B.

The term "sample of a bodily fluid" as used herein is a broad term and is to be given its ordinary and customary meaning to a person of ordinary skill in the art and is not to be limited to a special or customized meaning. The term specifically may refer, without limitation, to an arbitrary aliquot part or aliquant part of a biological fluid which directly is a bodily fluid or which is derived from a bodily fluid, such as by one or more pre-processing steps, e.g. by transferring a bodily fluid to at least one sampling fluid, by diluting a bodily fluid, by centrifugation a bodily fluid or the like. The bodily fluid may comprise one or more of saliva, blood, interstitial fluid, urine or other types of body fluids. The sample of the bodily fluid may be collected via at least one nasopharyngeal swab, at least one swab of the anterior nares or from saliva, such by applying a cotton swab to a surface of the anterior nares and/or the throat. The collected sample of bodily fluid may be transferred to at least sampling or reagent fluid by immersing the cotton swab in the sampling or reagent fluid. The sampling or reagent fluid may specifically comprise lysis reagents. Alternatively or additionally, the sample of the bodily fluid may be a droplet of a body fluid as gathered from the body of a person, such as a droplet of saliva and/or blood and/or interstitial fluid or the like. The sample of the bodily fluid may specifically comprise at least one preparation of the bodily fluid, such as a cell preparation of the bodily fluid, e.g. a stained cell preparation of the bodily fluid. The sample of bodily fluid may also be simply referred to as the sample or the test sample.

The method comprises using at least one first mobile device and at least one second mobile device, each of said mobile devices comprising at least one camera and at least one processor. The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone and/or a smartphone. Additionally or alternatively, the mobile device may also refer to a notebook, a tablet computer or another type of portable computer having at least one camera. Thus, generally, the first and second mobile devices may, independently from each other, be selected from the group consisting of: a cell phone having at least one camera, specifically a smart phone; a portable computer having at least one camera, specifically at least one of a notebook and a tablet computer. The terms "first" and "second" as used in the context of the first mobile device and the second mobile device are used for nomenclature only, specifically without ranking or numbering these items. Specifically, it may be possible to use one, two or even more first and/or second mobile devices. The first mobile device and the second mobile device may be embodied similar or even identical in construction or, alternatively, may be embodied different from each other. The first mobile device and the second mobile device specifically may be non-identical. As used herein, the term "non-identical" may refer to the fact that the first mobile device and the second mobile device may be two different entities or devices. However, the first mobile device and the second mobile device being non-identical may not exclude a similar embodiment or construction of both devices. Specifically, the first mobile device and the second mobile device, even though being non-identical, such as by being two different devices or entities, may be embodied similar or identical in construction, such as by having similar or even identical features and/or functions. However, also a different embodiment of the non-identical first and second mobile device may be feasible, such as the first and second mobile devices being different entities with differing constructions.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images. As used herein, without limitation, the term "image" specifically may relate to data recorded by using the camera, such as a plurality of electronic readings from the imaging device, such as the pixels of the camera chip.

The camera, besides the at least one camera chip or imaging chip, may comprise further elements, such as one or more optical elements, e.g. one or more lenses. As an example, the camera may be a fix-focus camera, having at least one lens which is fixedly adjusted with respect to the camera. Alternatively, however, the camera may also comprise one or more variable lenses which may be adjusted, automatically or manually. The invention specifically shall be applicable to cameras as usually used in mobile applications, such as notebook computers, tablets or, specifically, cell phones such as smartphones. Thus, specifically, the camera may be part of the mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B. A larger number of color values is also feasible, such as four colors for each pixel, for example R, G, G, B. Color cameras are generally known to the skilled person. Thus, as an example, each pixel of the camera chip may have three or more different color sensors, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B or C, M, Y, K or the like. The color sensitivities of the pixels may be generated by color filters or by appropriate intrinsic sensitivities of the sensor elements used in the camera pixels. These techniques are generally known to the skilled person.

The term "processor" as used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math co-processor or a numeric co-processor, a plurality of registers, specifically registers configured for supplying operands to the ALU and storing results of operations, and a memory, such as an L1 and L2 cache memory. In particular, the processor may be a multi-core processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) and/or one or more tensor processing unit (TPU) and/or one or more chip, such as a dedicated machine learning optimized chip, or the like. The processor specifically may be configured, such as by software programming, for performing one or more evaluation operations as will be outlined in further detail below.

The method further comprises using at least one optical test element comprising at least one reagent test region and at least one unique identifier associated with the optical test element.

The term "optical test element" as used herein is a broad term and is to be given its ordinary and customary meaning to a person of ordinary skill in the art and is not to be limited to a special or customized meaning. The term specifically may refer, without limitation, to an arbitrary element or device configured for performing an optical detection reaction, for example a color-change detection reaction and/or a reaction during which one or more optically detectable features on or within the test element become visible, such as one or more markings, such as linear markings known from rapid COVID testing. The optical test element may, as an example, be embodies as a test stick or as a test element. The optical test element may particularly have the at least one reagent test region containing at least one test chemical being sensitive for the property of the sample, such as for detecting the at least one analyte. The optical test element may, as an example, comprise one or more application sites for applying the at least one sample. The application site may be different from the position of the at least one reagent test region and may be fluidically connected to the reagent test region, such as by one or more capillary elements, such as one or more porous elements capable of transporting liquid. The optical test element, as an example, may comprise at least one substrate, such as at least one carrier, with the at least one reagent test region applied thereto or integrated therein. The optical test element may comprise at least one control area, specifically in a proximity to the reagent test region, for example enclosing or surrounding the reagent test region and/or arranged behind the reagent test region in a direction of flow of the sample of bodily fluid on the optical test element. The control area may be a separate field independently arranged on the substrate or carrier. The control area may be configured for indicating correct applying of the sample of the bodily fluid on the optical test element. The carrier, as an example, may be strip-shaped, thereby rendering the optical test element a test strip. These test strips are generally widely in use and available. One test strip may carry a single reagent rest region or a plurality of reagent test regions having identical or different test chemicals comprised therein. Additionally or alternatively, the optical test element may be embodied as a stick or chip, e.g. with a housing having the above-mentioned substrate disposed therein, e.g. a housing having one or more application openings for applying the sample and one or more detection windows for enabling an optical detection of the at least one detection reaction. Various options are feasible.

The optical test element may be a digital-type test element for digitally detecting the presence or absence of at least one predetermined analyte in the sample. Thus, the optical test element may be a test element capable of providing the information "positive" if the at least one predetermined analyte is determined to be present in the sample, or "negative" if the at least one predetermined analyte is determined not to be present in the sample. The optical test element may be configured for changing at least one optically detectable property of at least one feature when the analyte is detected in the sample, whereas the at least one optically detectable property of the at least one feature may be kept unchanged when the analyte is not detected in the sample. As an example, the optical test element may be a SARS-CoV-2 rapid antigen test, specifically a SARS-CoV-2 & Flu A/B rapid antigen test.

The term "reagent test region" as used herein is a broad term and is to be given its ordinary and customary meaning to a person of ordinary skill in the art and is not to be limited to a special or customized meaning. The term specifically may refer, without limitation, to a coherent amount of the test chemical, such as to a field, e.g. a field of round, polygonal or rectangular shape, having one or more layers of material, with at least one layer of the reagent test region having the test chemical comprised therein. Other layers may be present providing specific optical properties such as reflective properties, providing spreading properties for spreading the sample or providing separation properties such as for separating of particulate components of the sample, such as cellular components. The sample of the bodily fluid may be applied directly to the reagent test region, e.g. for blood glucose measurements where a droplet of blood may be directly applied to the test strip comprising the reagent test region, or may be applied indirectly to the reagent test region, e.g. by applying the sample of the bodily fluid to a reservoir or an application site of the optical test element, wherein the sample of the bodily fluid may flow from the reservoir of application site to the reagent test region of the optical test element, such as by capillary forces acting on the sample of the bodily fluid.

The term "unique identifier" as used herein is a broad term and is to be given its ordinary and customary meaning to a person of ordinary skill in the art and is not to be limited to a special or customized meaning. The term specifically may refer, without limitation, to an element or a combination of elements configured for storing one or more items of identity information identifying the optical test element, such as in a readable fashion, specifically in a machine-readable fashion. The unique identifier may comprise at least one of an optical identifier, an electronic identifier, a magnetic identifier or a mechanical identifier. As an example, the unique identifier, specifically the optical identifier, may be or may comprise at least one of a one- or two-dimensional code and/or a readable information tag, such as one or more of a barcode, a QR code or another type of code directly or indirectly attached to and/or integrated into the optical test element such as by being applied directly to the optical test element and/or by being attached to the optical test element via at least one label or tag. The item of identity information stored in the unique identifier may be derived using an appropriate reading device, such as by using the camera of the first and/or second mobile device. The unique identifier may also be configured for evaluating a validity of the assigned optical test element, such as by allowing checking expiry date, manufacturer and/or whether the optical test element has already been used or registered.

The method comprises the following steps that, as an example, may be performed in the given order. It shall be noted, however, that a different order may generally also be possible. Further, it may also be possible to perform one or more of the method steps once or repeatedly. Further, it may also be possible to perform two or more of the method steps simultaneously or in a timely overlapping fashion. The method may comprise further method steps that are not listed.

The method further comprises using at least one database, specifically at least one cloud-based database. The term "database" as used herein is a broad term and is to be given its ordinary and customary meaning to a person of ordinary skill in the art and is not to be limited to a special or customized meaning. The term specifically may refer, without limitation, to an organized collection of data, generally stored and accessed electronically from a computer or computer system. The database may comprise or may be comprised by a data storage device. The database may comprise at least one data base management system comprising a software running on a computer or computer system, the software allowing for interaction with one or more of a user, an application or the database itself, such as in order to capture and analyze the data contained in the data base. The database management system may further encompass facilities to administer the database. The database, containing the data, may, thus, be comprised by a database system which, besides the data, comprises one or more associated items of information and/or one or more associated applications. The database may specifically be a cloud-based database. The cloud-based database may have the data storage device independent from the first mobile device and the second mobile device and may be accessible via at least one data connection, specifically a wireless data connection.

The method, as defined in claim 1, comprises:
i. capturing at least one first image by using the camera of the first mobile device, before applying the sample of the bodily fluid to the reagent test region of the optical test element, said first image comprising at least a part of the unique identifier;
ii. storing at least one time stamp and at least one first item of identity information on the at least one optical test element in the database, wherein the at least one first item of identity information is derived from the unique identifier in the first image;
iii. capturing at least one second image by using the camera of the second mobile device, said second image comprising at least a part of the at least one unique identifier and at least a part of the reagent test region of the optical test element having the sample of the bodily fluid applied thereto;
iv. verifying, by using the database, if the unique identifiers in the first image and in the second image are identical and verifying if at least a predetermined period of minimum waiting time has elapsed since a point in time defined by the time stamp, wherein the method further comprises, based on the result of the verifyings, taking an action selected from the group of actions comprising:
   - aborting further evaluation of the respective optical test element if the unique identifiers in the first and second images are not identical;
   - aborting further evaluation of the respective optical test element if the predetermined period of minimum waiting time since the point in time defined by the time stamp has not yet elapsed;
   - aborting further evaluation of the respective optical test element if at least a predetermined period of maximum waiting time since the point in time defined by the time stamp has exceeded;
   - determining the at least one property of the sample by using at least the second image of the respective optical test element.

The term "capturing at least one image" as used herein is a broad term and is to be given its ordinary and customary meaning to a person of ordinary skill in the art and is not to be limited to a special or customized meaning. The term specifically may refer, without limitation, to one or more of imaging, image recording, image acquisition, image capturing. The term "capturing at least one image" may comprise capturing a single image and/or a plurality of images such as a sequence of images. For example, the capturing of the image may comprise recording continuously a sequence of images such as a video or a movie. The capturing of the at least one image may be initiated by the user action or may automatically be initiated, e.g. once the presence of the at least one object within a field of view and/or within a predetermined sector of the field of view of the camera is automatically detected. These automatic image acquisition techniques are known e.g. in the field of automatic bar-code readers, such as from automatic barcode reading apps. The capturing of the images may take place, as an example, by acquiring a stream or "live stream" of images with the camera, wherein one or more of the images, automatically or by user interaction such as pushing a button, are stored and used as the at least one first image or the at least one second image, respectively. The image acquisition may be supported by the processor of the first and/or second mobile device, and the storing of the images may take place in a data storage device of the first and/or second mobile device. Alternatively or additionally, the captured images may fully or partially be transferred to a cloud-based system and the storing of the images may fully or partially take place in a data storage device of the cloud-based system.

The term "first image" as used herein is a broad term and is to be given its ordinary and customary meaning to a person of ordinary skill in the art and is not to be limited to a special or customized meaning. The term specifically may refer, without limitation, to an image, as defined above, of the optical test element without having the sample of the bodily fluid applied to the reagent test region. Specifically, the first image may be at least one image of a new and/or unused optical test element. The first image comprises at least a part of the unique identifier, specifically at least a sufficient part of the unique identifier to derive the one or more items of identity information identifying the optical test element from the unique identifier.

The term "time stamp" as used herein is a broad term and is to be given its ordinary and customary meaning to a person of ordinary skill in the art and is not to be limited to a special or customized meaning. The term specifically may refer, without limitation, to an arbitrary array or sequence of characters, such as an array or a sequence of numbers and/or letters, indicating a point in time. Specifically, the time stamp may indicate a point in time for starting the predetermined period of minimum waiting time. Specifically, the time stamp may be used as a proxy for determining a point in time when the sample of the bodily fluid may be applied to the optical test element. For example, the time stamp may indicate a point in time when the predetermined period of minimum waiting time starts running.

The term "predetermined period of minimum waiting time" as used herein is a broad term and is to be given its ordinary and customary meaning to a person of ordinary skill in the art and is not to be limited to a special or customized meaning. The term specifically may refer, without limitation, to a time interval defining an amount of time between capturing the first image and the second image. The predetermined period of minimum waiting time may be defined prior to performing the analytical method. The predetermined period of minimum waiting time may be defined such that the optical detection reaction, for example the color-change detection reaction, at the optical test element, specifically at the reagent test region, has developed sufficiently to be detected. The predetermined period of minimum waiting time may define a minimum waiting time which has to elapse in order to ensure that the property of the sample can be reliably determined at the optical test element. The predetermined period of minimum waiting time may be smaller than the predetermined period of maximum waiting time after which the property cannot be reliably determined from the optical test element, for example due to artifacts affecting the optical detection reaction at the optical test element. The predetermined period of minimum waiting time may depend on the specific analyte of interest. For example, the analyte may be or may comprise the SARS-CoV-2 coronavirus. In this case, the predetermined period of minimum waiting time may be at least 15 minutes and, specifically, no more than 60 minutes, more specifically no more than 45 minutes, most specifically no more than 30 minutes.

The predetermined period of minimum waiting time, in step iv., specifically, may be any specific value in the range from 5 minutes to 60 minutes, specifically in the range from 10 minutes to 45 min, more specifically in the range from 15 minutes to 30 minutes, most specifically 15 minutes. The predetermined period of minimum waiting time may be started at the point in time defined by the time stamp, specifically at a point of time selected from an application time of the sample of the bodily fluid to the reagent test region and a point in time when the sample of the bodily fluid has been applied to the reagent test region. Specifically, the method may comprise prompting the user to apply the sample of the bodily fluid to the reagent test region. The point in time of the prompting may be indicative of the application time of the sample of the bodily fluid to the reagent test region and/or the point in time when the sample of the bodily fluid has been applied to the reagent test region. Alternatively or additionally, the user may be prompted to confirm application of the sample of the bodily fluid to the optical test element. The point in time of the confirmation by user may be used as the time stamp, specifically as the starting point for the predetermined period of minimum waiting time. For example, the method may comprise, specifically in step ii., prompting the user to apply the sample of the bodily fluid to the optical test element, specifically to the reagent test region. The application of the sample of the bodily fluid to the optical test element, specifically to the reagent test region, may determine a starting point for the predetermined period of minimum waiting time.

The term "item of identity information", used in the context of the first item of identity information and/or a second item of identity information, as used herein is a broad term and is to be given its ordinary and customary meaning to a person of ordinary skill in the art and is not to be limited to a special or customized meaning. The term specifically may refer, without limitation, to an item of information, such as an array or a sequence of numbers and/or letters, encoding identification information of a specific optical test element. The item of identity information may be unique for a specific optical test element. Thus, it may be possible to identify a specific optical test element using the respective item of identity information. For example, the item of identity information may comprise a test ID of the optical test element. The item of identity information may further comprise and/or may be associated with at least one of: an expiry date of the optical test element; a manufacturer of the optical test element; information whether the optical test element has already been used or registered.

The term "second image" as used herein is a broad term and is to be given its ordinary and customary meaning to a person of ordinary skill in the art and is not to be limited to a special or customized meaning. The term specifically may refer, without limitation, to an image, as defined above, of the optical test element having the sample of the bodily fluid applied to the reagent test region. Specifically, the second image may be captured after the predetermined period of minimum waiting time. The second image may be captured at a point in time where the optical detection reaction, for example the color-change detection reaction, at the optical test element, specifically at the reagent test region, has developed sufficiently to be detected, which may specifically be ensured by verifying that the predetermined period of minimum waiting time since the point in time defined by the time stamp has elapsed. The second image comprises at least a part of the unique identifier and at least a part of the reagent test region, specifically at least a sufficient part of the unique identifier to derive the one or more items of identity information identifying the optical test element from the unique identifier and at least a sufficient part of the reagent test region to evaluate the optical detection reaction.

The term "verifying" as used herein is a broad term and is to be given its ordinary and customary meaning to a person of ordinary skill in the art and is not to be limited to a special or customized meaning. The term specifically may refer to the process of establishing at least one item of information on the truth, accuracy, or reality of at least one statement, question or condition. Thus, the term specifically may refer, without limitation, to a process of checking two or more identifiers for their identity. The verifying may comprise comparing the unique identifiers in the first image and in the second image, specifically comparing the one or more items of identity information stored in the unique identifiers in the first image and in the second image, respectively. The verifying may comprise checking if the first item of identity information is stored in the database. In case that only non-identical items of identity information are found in the database for the second item of identity information, the respective second item of identity information may be flagged as being an invalid optical test element in order to prevent further use of this optical test element. The result of the verifying may be an item of information indicating the verified unique identifiers to be "identical" or "not identical". For example, if the one or more items of identity information derived from the unique identifier in the first image can also be derived from the unique identifier in the second image, the result of the verifying may be the item of information "identical". For example, if the one or more items of identity information derived from the unique identifier in the first image cannot be derived from the unique identifier in the second image, the result of the verifying may be the item of information "not identical".

The term "identical" as used herein, specifically in the context of the unique identifier, is a broad term and is to be given its ordinary and customary meaning to a person of ordinary skill in the art and is not to be limited to a special or customized meaning. The term specifically may refer, without limitation, to a situation where the compared unique identifiers comprise the same one or more items of identity information. Specifically, two or more unique identifiers may be assumed identical if the one or more items of identity information stored in one unique identifier can also be derived from the other unique identifiers.

Alternatively or additionally, the verifying may comprise checking if the predetermined period of minimum waiting time has elapsed since the point in time defined by the time stamp. For example, the verifying may comprise comparing a point in time when the second image was captured with a point in time defined by an elapse of the predetermined period of minimum waiting time started at a point in time defined by the time stamp. If the predetermined period of minimum waiting time has elapsed and the point in time indicating the capturing of the second image does not exceed the predetermined period of maximum waiting time since the point in time defined by the time stamp, the result of the verifying may be an item of information indicating the optical test element is ready for evaluation. If the predetermined period of minimum waiting time has not yet elapsed, the result of the verifying may an item of information indicating an insufficient waiting time, e.g. by prompting the user of the second mobile device the remaining waiting time until the predetermined period of minimum waiting time has elapsed. If the predetermined period of minimum waiting time has elapsed but the point in time indicating the capturing of the second image exceeds the predetermined period of maximum waiting time since the point in time defined by the time stamp, the result of the verifying may be an item of information indicating an invalid optical test element. In this case, the evaluation of the optical test element may be aborted. In case the optical test element is ready for evaluation, such as by having identical items of identity information and by having an elapsed predetermined period of minimum waiting time, the method may proceed with evaluation the optical test element and storing the determined property in the database. In this case, the test ID of the optical test element may be assigned with an item of information indicating a terminated evaluation, e.g. "test finished" or the like. Thus, it may be possible to automatically ensure proper handling of the optical test element and its evaluation.

Alternatively or additionally, the verifying may comprise checking if the predetermined period of maximum waiting time since the point in time defined by the time stamp has not exceeded. For example, the verifying may comprise comparing a point in time when the second image was captured with a point in time defined by an elapse of the predetermined period of maximum waiting time started at a point in time defined by the time stamp. If the predetermined period of maximum waiting time since the point in time defined by the time stamp has not yet exceeded and the predetermined period of minimum waiting time has elapsed, the result of the verifying may be an item of information indicating the optical test element is ready for evaluation. If the predetermined period of maximum waiting time since the point in time defined by the time stamp has exceeded, the result of the verifying may an item of information indicating an invalid optical test element and the evaluation of the invalid optical test element may be prevented.

The term "predetermined period of maximum waiting time" as used herein is a broad term and is to be given its ordinary and customary meaning to a person of ordinary skill in the art and is not to be limited to a special or customized meaning. The term specifically may refer, without limitation, to a time interval defining a maximum readout-time for the optical test element. Specifically, the maximum waiting may define a time interval beginning with sample application after which the property cannot be reliably determined from the optical test element, for example due to artifacts affecting the optical detection reaction at the optical test element. The predetermined period of maximum waiting time may be started at a point in time defined by the time stamp. The predetermined period of maximum waiting time may be 1 hour, specifically 2 hours, more specifically 5 hours, more specifically 10 hours, more specifically 24 hours, most specifically 48 hours or even more. The predetermined period of maximum waiting time may depend on the specific analyte of interest. For example, the analyte may be or may comprise the SARS-CoV-2 coronavirus. In this example, the predetermined period of maximum waiting time may be 60 minutes, specifically 45 min, more specifically 30 minutes.

The method comprises, as outlined above, storing the time stamp and the at least one first item of identity information on the at least one optical test element in the database. The at least one first item of identity information is derived from the unique identifier in the first image. Additionally, the first image captured in step i. and/or image acquisition settings of the camera of the first mobile device may be stored in the database. Additionally or alternatively, the database may comprise information on a state of the optical test element associated with the first item of identity information. For example, the database may comprise an information "test started", specifically if the first image was captured, the sample of the bodily fluid was applied to the optical test element and the time stamp was stored. For example, the database may comprise an information "test finished", specifically if the second image was captured, the optical test element was successfully evaluated and the corresponding property of the sample was stored. For example, the database may comprise an information "test invalid", specifically if the predetermined period of maximum waiting time for capturing the second image has expired or if the second image was captured without having stored the information "test started" for the respective optical test element in the database. For example, the database may comprise an information "test expired", specifically if the first image was captured but an expiry date of the optical test element has expired.

Step ii. may comprise, specifically prior to the storing of the time stamp and the first item of identity information, checking if the optical test element has already been used. For example, step ii. may comprise checking if the first item of identity information has already been stored in the database. If the first item of identity information is already stored in the database, the respective optical test element may be classified as being used and the additional fist image may be denied. If the first item of identity information is not stored in the database, the respective optical test element may be classified as being unused and the method may proceed by storing the time stamp and the respective first item of identity information, e.g. by creating a new database entry.

The method, in step iv., may comprise deriving at least one second item of identity information on the at least one optical test element from the unique identifier in the second image. The verifying if the unique identifiers in the first image and in the second image are identical in step iv. may comprise verifying if the second item of identity information is identical with at least one of the first items of identity information in the database.

The method may further comprise in step iv. retrieving the time stamp and the item of identity information on the at least one optical test element from the database. Specifically, the using of the database in step iv. may comprise retrieving the time stamp and the item of identity information on the at least one optical test element from the database.

The method may further comprise repeating steps iii. and iv. in case the further evaluation of the respective optical test element is aborted due to non-identical unique identifiers in the first and second images or due to an unelapsed predetermined period of minimum waiting time since the point in time defined by the time stamp. The method may comprise providing at least one notification using the second mobile device. The notification may be one or more of a visual notification, a haptic notification and/or an acoustic notification. Specifically, the method may comprise displaying the notification via at least one display device of the second mobile device. The notification may comprise prompting a user of the second mobile device to repeat performing step iii..

The analytical method may comprise using a plurality of test samples and a plurality of optical test elements. Each test sample may be assigned to an optical test element. In the method:
- step i. may comprise capturing at least one first image for each of the optical test elements, thereby generating a set of first images;
- step iii. may comprise capturing at least one second image for each of the optical test elements, thereby generating a set of second images; and
- step iv. may comprise verifying, for each of the second images of the set of second images, if the unique identifier in the respective second image is also present in at least one first image of the set of first images.

Further, the verifying in step iv. may be performed for each of the optical test elements individually and independently.

The analytical method may comprise mass-testing of a plurality of samples in a test center, specifically in at least one of a public test center and a test center in a hospital. The term "mass-testing" as used herein is a broad term and is to be given its ordinary and customary meaning to a person of ordinary skill in the art and is not to be limited to a special or customized meaning. The term specifically may refer, without limitation, to a multiple performance of the analytical method. The mass-testing may comprise performing the analytical method, specifically steps i. to iv., multiple times, specifically at least two times, more specifically at least five times, even more specifically at least ten times or even more. As an example, the mas testing may comprise performing the analytical method, specifically steps i. to iv., multiple times per day, specifically at least two times per day, more specifically at least five times per day, even more specifically at least ten times per day or even more often.

The steps i. to iv. may be performed at least once with a given sample from one user or patient. The mass-testing may specifically comprise performing the analytical method with the plurality of samples, wherein the plurality of samples may, for example, be collected from a plurality of different users or patients. The mass-testing may specifically comprise performing the steps i. to iv. for the plurality of samples in parallel or at least in a timely-overlapping fashion.

In a further aspect of the present invention, as defined in claim 5, a method of operating the first mobile device is disclosed. The method of operating the first mobile device specifically may be used in or may be part of the analytical method as discussed above or as discussed in further detail below. The first mobile device has the features as defined with respect to the analytical method according to the present invention. The method comprises the first mobile device performing at least steps i. and ii. of the analytical method according to the present invention, such as according to any one of the embodiments disclosed above and/or according to any one of the embodiments disclosed in further detail below. The method further comprises, specifically prior to step ii., the processor of the first mobile device deriving at least one first item of identity information on the at least one optical test element from the unique identifier in the first image.

For definitions of terms and possible embodiments of the method of operating the first mobile device, reference is made to the description of the analytical method as outlined above and/or as outlined in further detail below.

In a further aspect of the present invention, as defined in claim 7, a method of operating the second mobile device is disclosed. The method of operating the second mobile device specifically may be used in or may be part of the analytical method as discussed above or as discussed in further detail below. The second mobile device has the features as defined with respect to the analytical method according to the present invention. The method comprises performing at least steps iii. and iv. of the analytical method according to the present invention, such as according to any one of the embodiments disclosed above and/or according to any one of the embodiments disclosed in further detail below. The method further comprises the processor of the second mobile device, in step iv., deriving at least one second item of identity information on the at least one optical test element from the unique identifier in the second image. The method further comprises the processor of the second mobile device, in step iv., verifying if the second item of identity information is identical with at least one of first items of identity information in the database having stored therein a plurality of first items of identity information by using the method of operating the first mobile device according to the present invention, such as according to any one of the embodiments disclosed above and/or according to any one of the embodiments disclosed in further detail below.

For definitions of terms and possible embodiments of the method of operating the second mobile device, reference is made to the description of the analytical method as outlined above and/or as outlined in further detail below.

In a further aspect of the present invention, as defined in claim 8, a computer program is disclosed, comprising instructions which, when the program is executed by the processor of the first mobile device with the features as defined in the analytical method according to the present invention, such as according to any one of the embodiments disclosed above and/or according to any one of the embodiments disclosed in further detail below, cause the processor to control the first mobile device for performing the method of operating the first mobile device according to the present invention, such as according to any one of the embodiments disclosed above and/or according to any one of the embodiments disclosed in further detail below.

Similarly, as defined in claim 9, a computer-readable storage medium, specifically a non-transient storage medium, is disclosed, comprising instructions which, when the instructions are executed by the processor of the first mobile device with the features as defined in the analytical method according to the present invention, such as according to any one of the embodiments disclosed above and/or according to any one of the embodiments disclosed in further detail below, cause the processor to control the first mobile device for performing the method of operating the first mobile device according to the present invention, such as according to any one of the embodiments disclosed above and/or according to any one of the embodiments disclosed in further detail below.

As used herein, the term "computer-readable storage medium" specifically may refer to non-transitory data storage means, such as a hardware storage medium having stored thereon computer-executable instructions. The computer-readable storage medium specifically may be or may comprise a storage medium such as a random-access memory (RAM) and/or a read-only memory (ROM).

In a further aspect of the present invention, as defined in claim 10, a computer program is disclosed, comprising instructions which, when the program is executed by the processor of the second mobile device with the features as defined in the analytical method according to the present invention, such as according to any one of the embodiments disclosed above and/or according to any one of the embodiments disclosed in further detail below, cause the processor to control the second mobile device for performing the method of operating the second mobile device according to the present invention, such as according to any one of the embodiments disclosed above and/or according to any one of the embodiments disclosed in further detail below.

Similarly, as defined in claim 11, a computer-readable storage medium, specifically a non-transient storage medium, is disclosed, comprising instructions which, when the instructions are executed by the processor of the second mobile device with the features as defined in the analytical method according to the present invention, such as according to any one of the embodiments disclosed above and/or according to any one of the embodiments disclosed in further detail below, cause the processor to control the second mobile device for performing the method of operating the second mobile device according to the present invention, such as according to any one of the embodiments disclosed above and/or according to any one of the embodiments disclosed in further detail below.

In a further aspect of the present invention, as defined in claim 12, a first mobile device is disclosed, comprising at least one camera and at least one processor. The first mobile device is configured for performing the method of operating the first mobile device according to the present invention, such as according to any one of the embodiments disclosed above and/or according to any one of the embodiments disclosed in further detail below.

For definitions of terms and possible embodiments of the first mobile device, reference is made to the description of the analytical method as outlined above and/or as outlined in further detail below.

In a further aspect of the present invention, as defined in claim 13, a second mobile device is disclosed, comprising at least one camera and at least one processor. The second mobile device is configured for performing the method of operating the second mobile device according to the present invention, such as according to any one of the embodiments disclosed above and/or according to any one of the embodiments disclosed in further detail below.

For definitions of terms and possible embodiments of the first mobile device, reference is made to the description of the analytical method as outlined above and/or as outlined in further detail below.

In a further aspect of the present invention, as defined in claim 14, an analytical system is disclosed, comprising at least one first mobile device, specifically at least one first mobile device according to the present invention, such as according to any one of the embodiments disclosed above and/or according to any one of the embodiments disclosed in further detail below, and at least one second mobile device, specifically at least one second mobile device according to the present invention, such as according to any one of the embodiments disclosed above and/or according to any one of the embodiments disclosed in further detail below. Each of the first and second mobile devices comprises at least one camera and at least one processor. The analytical system further comprises at least one optical test element comprising at least one reagent test region and at least one unique identifier associated with the optical test element. The analytical system further comprises at least one database, specifically at least one cloud-based database. The analytical system is configured for performing the analytical method according to the present invention, such as according to any one of the embodiments disclosed above and/or according to any one of the embodiments disclosed in further detail below.

For definitions of terms and possible embodiments of the analytical system, reference is made to the description of the analytical method as outlined above.

The term "system" as used herein is a broad term and is to be given its ordinary and customary meaning to a person of ordinary skill in the art and is not to be limited to a special or customized meaning. The term specifically may refer, without limitation, to an arbitrary set of interacting or interdependent components parts forming a whole. The system may comprise multiple components, for example at least two components or even more. The at least two components may be handled independently or may be coupled or connectable. The components of the system may interact with each other in order to fulfill at least one common function. Thus, as used herein, the term "analytical system", may refer to a system for performing at least one analytical function, specifically for performing at least one analytical measurement, for example at least one analytical measurement as described above.

In a further aspect of the present invention, as defined in claim 15, a computer program is disclosed, comprising instructions which, when the program is executed by the analytical system according to the present invention, such as according to any one of the embodiments disclosed above and/or according to any one of the embodiments disclosed in further detail below, cause the analytical system to perform the analytical method according to the present invention, such as according to any one of the embodiments disclosed above and/or according to any one of the embodiments disclosed in further detail below.

Similarly, as defined in claim 16, a computer-readable storage medium, specifically a non-transient storage medium, is disclosed, comprising instructions which, when the instructions are executed by the analytical system according to the present invention, such as according to any one of the embodiments disclosed above and/or according to any one of the embodiments disclosed in further detail below, cause the analytical system to perform the analytical method according to the present invention, such as according to any one of the embodiments disclosed above and/or according to any one of the embodiments disclosed in further detail below.

The methods and devices according to the present invention may provide a large number of advantages compared with known methods and devices. Specifically, by using the methods and devices according to the present invention, it may be possible to avoid a strict organizational assignment of optical test elements to be analyzed and executing staff. By using two different mobile devices for the evaluation of the optical test element, the assignment can be avoided. Additionally, handling errors can be reduced and/or the overall throughput of measurements can be increased since two mobile devices provide more flexibility in evaluation of the optical test elements.

The analytical method may specifically be implemented in a photo app on mobile device, such as on a smartphone. The first and the second image of the optical test element, for example a lateral flow test assay, such as those used for rapid antigen tests for detecting SARS-CoV-2 coronavirus, may be captured by using the camera of the mobile device. From optically detectable changes within the reagent test region, such as an appearance of graphical elements, e.g. bars, dots or other geometric elements, or a specific color formation of the reagent test region, the presence or absence of the analyte and/or an analyte concentration value may be determined. For example, in a SARS-CoV-2 coronavirus test setting, such as in a large public test center, each individual optical test element may have the at least one unique identifier, which may be scanned by the photo app and may be uploaded in a cloud-based database in order to ensure a valid optical test element, for example by checking expiry date, manufacturer and/or whether the optical test element has already been used or registered. The first image, specifically providing an initial or blank scan, and the second image, specifically providing a final scan after a certain reaction time, may be captured using different mobile devices, specifically different smartphones, and the validity info necessary for the second image may be retrieved from the cloud-based database.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an embodiment of an analytical system comprising at least one first mobile device and at least one second mobile device;
- Figure 2: shows a flow chart of an embodiment of an analytical method of determining at least one property of at least one sample of a bodily fluid; and
- Figure 3: shows a flow chart of embodiments of a method of operating the first mobile device and a method of operating the second mobile device.

### Detailed description of the embodiments

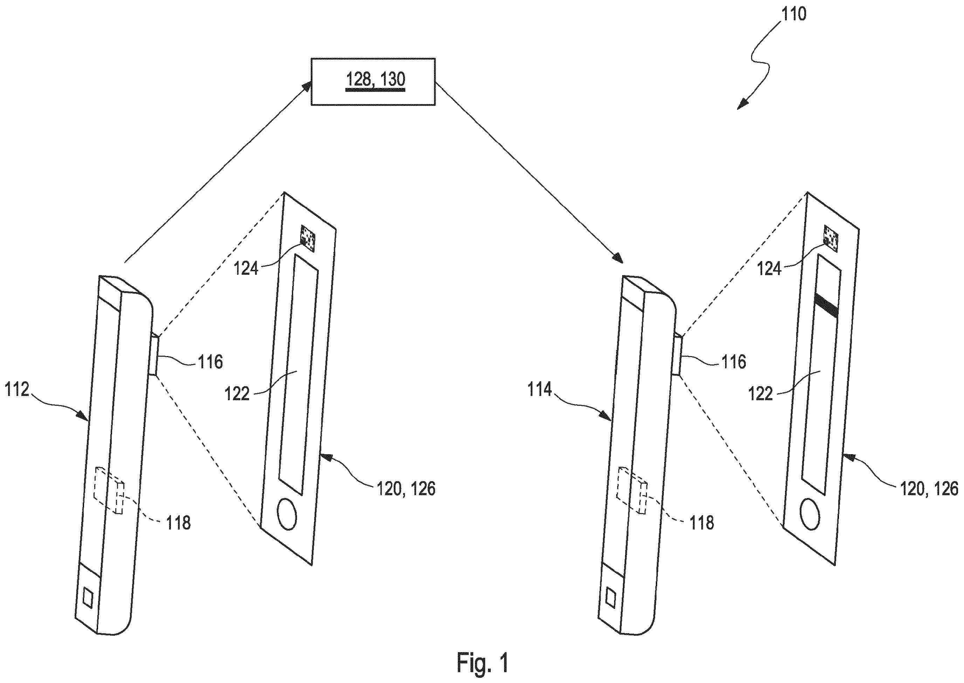

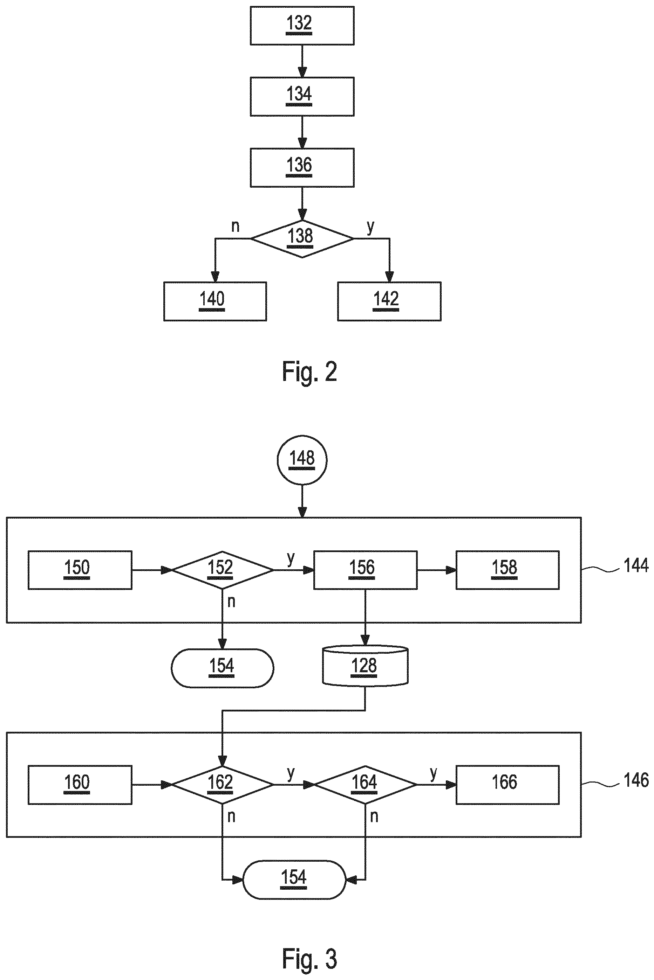

Figure 1 shows an exemplary embodiment of an analytical system 110 comprising at least one first mobile device 112 and at least one second mobile device 114. Each of the first mobile device 112 and the second mobile device 114 comprises at least one camera 116 and at least one processor 118. As can be seen in Figure 1, the first mobile device 112 and the second mobile device 114 not necessarily may be the same entity and, specifically, may be non-identical, specifically may be two different entities of mobile devices. The analytical system 110 may specifically comprise the first mobile device 112 being configured for performing a method of operating the first mobile device 112 according to the present invention, such as according to the exemplary embodiment shown in Figure 3 and/or according to any other embodiment disclosed herein. The analytical system 110 may specifically comprise the second mobile device 114 being configured for performing the method of operating the second mobile device 114 according to the present invention, such as according to the exemplary embodiment shown in Figure 3 and/or according to any other embodiment disclosed herein.

The analytical system 110 further comprises at least one optical test element 120 comprising at least one reagent test region 122 and at least one unique identifier 124 associated with the optical test element 120. In the example of Figure 1, the optical test element 120 may be at least one lateral flow test assay 126, such as those used for rapid antigen tests for detecting SARS-CoV-2 coronavirus. Thus, in this example, a property of a sample to be determined by the analytical system 110 may be the presence of at least one predetermined type of RNA virus, specifically the presence of the SARS-CoV-2 coronavirus. The optical test element 120 may specifically be a digital-type test element for digitally detecting the presence of the SARS-CoV-2 coronavirus. The optical test element 120 may be configured for changing at least one optically detectable property of at least one feature when the SARS-CoV-2 coronavirus is detected in the sample.

The analytical system 110 further comprises at least one database 128. The database 128 may specifically be a cloud-based database 130. As shown in Figure 1, the first mobile device 112 and the second mobile device 114 may be configured for exchanging information with the database 128, such as by storing information in the database 128 and/or by retrieving information from the database 128.

The analytical system 110 is configured for performing an analytical method according to present invention, such as according to the exemplary embodiment shown in Figure 2 and/or according to any other embodiment of the analytical method disclosed herein. Thus, for a description of the analytical method, reference is made to the description of Figure 2.

Figure 2 shows a flow chart of an exemplary first embodiment of the analytical method of determining at least one property of at least one sample of a bodily fluid. Specifically, the analytical method may be configured for detecting at least one analyte in the bodily fluid, e.g. for detecting the presence of the SARS-CoV-2 coronavirus in the sample of the bodily fluid.

The method comprises using the at least one first mobile device 112 and the at least one second mobile device 114, as exemplarily shown in Figure 1. Each of said mobile devices 112, 114 comprises the at least one camera 116 and the at least one processor 118. The method further comprises using the at least one optical test element 120 comprising the at least one reagent test region 122 and the at least one unique identifier 124 associated with the optical test element 120. The method further comprises using the at least one database 128, specifically the at least one cloud-based database 130.

The method comprises the following steps are to be performed in the given order. It shall be noted, however, that according to examples outside the claimed invention, a different order may generally also be possible.

Further, it may also be possible to perform one or more of the method steps once or repeatedly. Further, it may also be possible to perform two or more of the method steps simultaneously or in a timely overlapping fashion. The method may comprise further method steps that are not listed.

The method comprises:
i. (denoted by reference number 132) capturing at least one first image by using the camera 116 of the first mobile device 112, before applying the sample of the bodily fluid to the reagent test region 122 of the optical test element 120, said first image comprising at least a part of the unique identifier 124;
ii. (denoted by reference number 134) storing at least one time stamp and at least one first item of identity information on the at least one optical test element in the database, wherein the at least one first item of identity information is derived from the unique identifier in the first image;
iii. (denoted by reference number 136) capturing at least one second image by using the camera 116 of the second mobile device 114, said second image comprising at least a part of the at least one unique identifier 124 and at least a part of the reagent test region 122 of the optical test element 120 having the sample of the bodily fluid applied thereto;
iv. (denoted by reference number 138) verifying, by using the database 128, if the unique identifiers 124 in the first image and in the second image are identical and verifying if at least a predetermined period of minimum waiting time has elapsed since a point in time defined by the time stamp, wherein the method further comprises, based on the result of the verifyings, taking an action selected from the group of actions comprising:
   - (denoted by reference number 140) aborting further evaluation of the respective optical test element 120 if the unique identifiers 124 in the first and second images are not identical;
   - (denoted by reference number 140) aborting further evaluation of the respective optical test element 120 if the predetermined period of minimum waiting time since the point in time defined by the time stamp has not yet elapsed;
   - (denoted by reference number 140) aborting further evaluation of the respective optical test element 120 if at least a predetermined period of maximum waiting time since the point in time defined by the time stamp has exceeded;
   - (denoted by reference number 142) determining the at least one property of the sample by using at least the second image of the respective optical test element 120.

Specifically, the analytical method may comprise using a plurality of test samples and a plurality of optical test elements 120, wherein each test sample is assigned to an optical test element 120. In this example, step i. may comprise capturing the at least one first image for each of the optical test elements 120, thereby generating a set of first images. Step iii. may comprise capturing the at least one second image for each of the optical test elements 120, thereby generating a set of second images. Step iv. may comprise verifying, for each of the second images of the set of second images, if the unique identifier 124 in the respective second image is also present in at least one first image of the set of first images. The verifying in step iv. may specifically be performed for each of the optical test elements 120 individually and independently.

Figure 3 shows a flow chart of exemplary embodiments of a method of operating the first mobile device 112 and a method of operating the second mobile device 114. Specifically, the flow chart of Figure 3 shows a combined flow chart of embodiments of the method of operating the first mobile device 112 (denoted by reference number 144) and the method of operating the second mobile device 114 (denoted by reference number 146). The first mobile device 112 and the second mobile device 114 may be embodied as shown in Figure 1, respectively. Thus, for a description of the first mobile device 112 and the second mobile device 114, reference is made to the description of Figure 1.

As shown in Figure 3, the method of operating the first mobile device 112 may be initiated by starting the measurement sequence (denoted by reference number 148). The method of operating the first mobile device 112 comprises the first mobile device 112 performing at least step i. of the analytical method according to the present invention, such as according to the exemplary embodiment shown in Figure 2 and/or according to any other embodiment disclosed herein, i.e. the capturing the at least one first image by using the camera 116 of the first mobile device 112 (denoted by reference number 150). The method further comprises the processor 118 of the first mobile device 112 deriving at least one first item of identity information on the at least one optical test element 120 from the unique identifier 124 in the first image (denoted by reference number 152). The deriving of the first item of identity information may also comprise checking a validity of the optical test element 120 based on the derived first item of identity information, for example checking one or more of an expiry date of the optical test element 120, a manufacturer and whether the optical test element 120 has been used or registered already. In case this validity check reveals an invalid optical test element 120, the processor 118 of the first mobile device 112 may abort the method (denoted by reference number 154). In case the optical test element 120 is checked as being valid, the method may proceed by registering the optical test element 120 (denoted by reference number 156). The method further comprises performing step ii., i.e. the storing of the time stamp and the at least one first item of identity information in the at least one database 128 (denoted by reference number 158), specifically storing a point of time determining the beginning of the predetermined period of minimum waiting time of the analytical method. For example, the time stamp may indicate an application time of the sample of the bodily fluid to the reagent test region 122. Specifically, the information stored in the database 128 may comprise the at least first item of identity information and the at least one time stamp, such as the point in time determining the starting point for the predetermined period of minimum waiting time. Additionally, the first image captured in step i. and/or image acquisition settings of the camera 116 of the first mobile device 112 may be stored in the database 128.

As can be seen in Figure 3, the method of operating the second mobile device 114 comprises performing steps iii. of the analytical method according to the present invention, such as according to the exemplary embodiment shown in Figure 2 and/or according to any other embodiment disclosed herein, i.e. the capturing the at least one second image by using the camera 116 of the second mobile device 114 (denoted by reference number 160). Further, the method of operating the second mobile device 114 comprises performing step iv. of the analytical method according to the present invention, such as according to the exemplary embodiment shown in Figure 2 and/or according to any other embodiment disclosed herein, i.e. the verifying if the unique identifiers 124 in the first image and in the second image are identical (denoted by reference number 162). The method comprises the processor 118 of the second mobile device 114, in step iv., deriving at least one second item of identity information on the at least one optical test element 120 from the unique identifier 124 in the second image and verifying if the second item of identity information is identical with the at least one of first items of identity information in the database 128 having stored therein a plurality of first items of identity information by using the method of operating the first mobile device 112 as outlined above. In case the first and second items of identity information are not identical, the processor 118 of the second mobile device 114 may abort the method (denoted by reference number 154). In case the first and second items of identity information are identical, the method may further comprise the processor 118 of the second mobile device 114 determining if the predetermined period of minimum waiting time since the point in time defined by the time stamp has elapsed, specifically determining if the optical test element 120 is ready for measurement (denoted by reference number 164). In case the predetermined period of minimum waiting time since the point in time defined by the time stamp has not yet elapsed, the processor 118 of the second mobile device 114 may abort the method (denoted by reference number 154). The abort of the method in step 154 may specifically be a temporary abort. Specifically, the method may further comprise repeating steps iii. and iv. in case the further evaluation of the respective optical test element 120 is aborted due to non-identical unique identifiers 124 in the first and second images or due to an unelapsed predetermined period of minimum waiting time since the point in time defined by the time stamp. For example, the method may comprise providing at least one notification using the second mobile device 114, such as displaying the notification via at least one display device of the second mobile device 114. The notification may comprise prompting a user of the second mobile device 114 to repeat performing step iii..

In case the predetermined period of minimum waiting time since the point in time defined by the time stamp has elapsed, the method may further comprise determining the at least one property of the sample by using at least the second image of the respective optical test element 120 (denoted by reference number 166). However, in case the predetermined period of maximum waiting time since the point in time defined by the time stamp has exceeded, the method may comprise aborting further evaluation of the respective optical test element 120.

In this case, the abort of the method may be final and the respective optical test element 120 may be flagged as being invalid.

### List of reference numbers

- 110: analytical system
- 112: first mobile device
- 114: second mobile device
- 116: camera
- 118: processor
- 120: optical test element
- 122: reagent test region
- 124: unique identifier
- 126: lateral flow test assay
- 128: database
- 130: cloud-based database
- 132: capturing the first image
- 134: storing the time stamp and the first item of identity information on the optical test element in the database
- 136: capturing the second image
- 138: verifying the unique identifiers
- 140: aborting further evaluation
- 142: determining the at least one property of the sample
- 144: method of operating the first mobile device
- 146: method of operating the second mobile device
- 148: starting the measurement sequence
- 150: capturing the first image
- 152: deriving the first item of identity information
- 154: aborting the method
- 156: registering the optical test element
- 158: storing of the time stamp and the first item of identity information in the database
- 160: capturing the second image
- 162: verifying the unique identifiers
- 164: determining if the predetermined period of minimum waiting time has elapsed
- 166: determining the property of the sample

## Claims

1. An analytical method of determining at least one property of at least one sample of a bodily fluid, the method using at least one first mobile device (112) and at least one second mobile device (114), each of said mobile devices (112, 114) comprising at least one camera (116) and at least one processor (118), the method further using at least one optical test element (120) comprising at least one reagent test region (122) and at least one unique identifier (124) associated with the optical test element (120), the method further comprising using at least one database (128), the method comprising:
i. capturing at least one first image by using the camera (116) of the first mobile device (112), before applying the sample of the bodily fluid to the reagent test region (122) of the optical test element (120), said first image comprising at least a part of the unique identifier (124);
ii. storing at least one time stamp and at least one first item of identity information on the at least one optical test element (120) in the database (128), wherein the at least one first item of identity information is derived from the unique identifier (124) in the first image;
iii. capturing at least one second image by using the camera (116) of the second mobile device (114), said second image comprising at least a part of the at least one unique identifier (124) and at least a part of the reagent test region (122) of the optical test element (120) having the sample of the bodily fluid applied thereto;
iv. verifying, by using the database (128), if the unique identifiers (124) in the first image and in the second image are identical and verifying if at least a predetermined period of minimum waiting time has elapsed since a point in time defined by the time stamp, wherein the method further comprises, based on the result of the verifyings, taking an action selected from the group of actions comprising:
- aborting further evaluation of the respective optical test element (120) if the unique identifiers (124) in the first and second images are not identical;
- aborting further evaluation of the respective optical test element (120) if the predetermined period of minimum waiting time since the point in time defined by the time stamp has not yet elapsed;
- aborting further evaluation of the respective optical test element (120) if at least a predetermined period of maximum waiting time since the point in time defined by the time stamp has exceeded;
- determining the at least one property of the sample by using at least the second image of the respective optical test element (120).

2. The analytical method according to the preceding claim, wherein the method, in step iv., comprises deriving at least one second item of identity information on the at least one optical test element (120) from the unique identifier (124) in the second image, wherein the verifying if the unique identifiers (124) in the first image and in the second image are identical comprises verifying if the second item of identity information is identical with at least one of the first items of identity information in the database (128).

3. The analytical method according to any one of the preceding claims, wherein the analytical method comprises using a plurality of test samples and a plurality of optical test elements (120), wherein each test sample is assigned to an optical test element (120), wherein
- step i. comprises capturing at least one first image for each of the optical test elements (120), thereby generating a set of first images;
- step iii. comprises capturing at least one second image for each of the optical test elements (120), thereby generating a set of second images; and
- step iv. comprises verifying, for each of the second images of the set of second images, if the unique identifier (124) in the respective second image is also present in at least one first image of the set of first images;
wherein, optionally, the verifying in step iv. is performed for each of the optical test elements (120) individually and independently.

4. The analytical method according to any one of the preceding claims, wherein the method comprises mass-testing of a plurality of samples in a test center.

5. A method of operating the first mobile device (112) with the features as defined in claim 1, the method comprising the first mobile device (112) performing at least steps i. and ii. of the method according to any one of the preceding claims referring to an analytical method, the method further comprising the processor (118) of the first mobile device (112) deriving the at least one first item of identity information on the at least one optical test element (120) from the unique identifier (124) in the first image, wherein the database (128) is a cloud-based database, wherein the cloud-based database has a data storage device independent from the first mobile device (112) and the second mobile device (114) and is accessible via at least one data connection.

6. The method according to the preceding claim, wherein step ii. comprises, prior to the storing of the time stamp and the first item of identity information, checking if the optical test element (120) has already been used, wherein step ii. comprises checking if the first item of identity information has already been stored in the database (128), wherein, if the first item of identity information is already stored in the database (128), the respective optical test element (120) is classified as being used and the first image is denied, wherein, if the first item of identity information is not stored in the database (128), the respective optical test element (120) is classified as being unused and the method proceeds by storing the time stamp and the respective first item of identity information.

7. A method of operating the second mobile device (114) with the features as defined in claim 1, the method comprising performing at least steps iii. and iv. of the method according to any one of the preceding claims referring to an analytical method, the method further comprising the processor (118) of the second mobile device (114), in step iv., deriving at least one second item of identity information on the at least one optical test element (120) from the unique identifier (124) in the second image, the method further comprising the processor (118) of the second mobile device (114), in step iv., verifying if the second item of identity information is identical with at least one of first items of identity information in the database (128) having stored therein a plurality of first items of identity information by using the method according to the preceding claim.

8. A computer program comprising instructions which, when the program is executed by the processor (118) of the first mobile device (112) with the features as defined in claim 1, cause the processor (118) to control the first mobile device (112) for performing the method according to claim 5.

9. A computer-readable storage medium comprising instructions which, when the instructions are executed by the processor (118) of the first mobile device (112) with the features as defined in claim 1, cause the processor (118) to control the first mobile device (112) for performing the method according to claim 5.

10. A computer program comprising instructions which, when the program is executed by the processor (118) of the second mobile device (114) with the features as defined in claim 1, cause the processor (118) to control the second mobile device (114) for performing the method according to claim 7.

11. A computer-readable storage medium comprising instructions which, when the instructions are executed by the processor (118) of the second mobile device (114) with the features as defined in claim 1, cause the processor (118) to control the second mobile device (114) for performing the method according to claim 7.

12. A first mobile device (112) comprising at least one camera (116) and at least one processor (118), wherein the first mobile device (112) is configured for performing the method according to claim 5.

13. A second mobile device (114) comprising at least one camera (116) and at least one processor (118), wherein the second mobile device (114) is configured for performing the method according to claim 7.

14. An analytical system (110) comprising at least one first mobile device (112) and at least one second mobile device (114), wherein each of the first (112) and second mobile devices (114) comprises at least one camera (116) and at least one processor (118), the analytical system (110) further comprising at least one optical test element (120) comprising at least one reagent test region (122) and at least one unique identifier (124) associated with the optical test element (120), the analytical system (110) further comprising at least one database (128), wherein the analytical system (110) is configured for performing the analytical method according to any one of the preceding claims referring to an analytical method.

15. A computer program comprising instructions which, when the program is executed by the analytical system (110) according to claim 14, cause the analytical system (110) to perform the analytical method according to any one of the preceding claims referring to an analytical method.

16. A computer-readable storage medium comprising instructions which, when the instructions are executed by the analytical system (110) according to claim 14, cause the analytical system (110) to perform the analytical method according to any one of the preceding claims referring to an analytical method.

## Patentansprüche

1. Analytisches Verfahren zum Bestimmen mindestens einer Eigenschaft mindestens einer Probe einer Körperflüssigkeit, wobei das Verfahren mindestens eine erste mobile Vorrichtung (112) und mindestens eine zweite mobile Vorrichtung (114) verwendet, wobei jede der mobilen Vorrichtungen (112, 114) mindestens eine Kamera (116) und mindestens einen Prozessor (118) umfasst, wobei das Verfahren ferner mindestens ein optisches Testelement (120) verwendet, das mindestens einen Reagenztestbereich (122) und mindestens eine eindeutige Kennung (124) umfasst, die dem optischen Testelement (120) zugeordnet ist, wobei das Verfahren ferner das Verwenden mindestens einer Datenbank (128) umfasst, wobei das Verfahren Folgendes umfasst:
i. Aufnehmen mindestens eines ersten Bildes unter Verwendung der Kamera (116) der ersten mobilen Vorrichtung (112), bevor die Probe der Körperflüssigkeit auf den Reagenztestbereich (122) des optischen Testelements (120) aufgebracht wird, wobei das erste Bild mindestens einen Teil der eindeutigen Kennung (124) umfasst;
ii. Speichern mindestens eines Zeitstempels und mindestens eines ersten Elements von Identitätsinformationen auf dem mindestens einen optischen Testelement (120) in der Datenbank (128), wobei das mindestens eine erste Element von Identitätsinformationen von der eindeutigen Kennung (124) in dem ersten Bild abgeleitet ist;
iii. Aufnehmen mindestens eines zweiten Bildes unter Verwendung der Kamera (116) der zweiten mobilen Vorrichtung (114), wobei das zweite Bild mindestens einen Teil der mindestens einen eindeutigen Kennung (124) und mindestens einen Teil des Reagenztestbereichs (122) des optischen Testelements (120) umfasst, auf den die Probe der Körperflüssigkeit aufgebracht wurde;
iv. Verifizieren, unter Verwendung der Datenbank (128), ob die eindeutigen Kennungen (124) in dem ersten Bild und in dem zweiten Bild identisch sind, und Verifizieren, ob mindestens eine vorbestimmte Dauer von minimaler Wartezeit seit einem Zeitpunkt verstrichen ist, der durch den Zeitstempel definiert ist, wobei das Verfahren ferner, basierend auf dem Ergebnis der Verifizierungen, das Durchführen einer Aktion umfasst, ausgewählt aus der Gruppe von Aktionen, die Folgendes umfasst:
- Abbrechen der weiteren Auswertung des jeweiligen optischen Testelements (120), wenn die eindeutigen Kennungen (124) in dem ersten und dem zweiten Bild nicht identisch sind;
- Abbrechen der weiteren Auswertung des jeweiligen optischen Testelements (120), wenn die vorbestimmte Dauer einer minimalen Wartezeit seit dem Zeitpunkt, der durch den Zeitstempel definiert ist, noch nicht verstrichen ist;
- Abbrechen der weiteren Auswertung des jeweiligen optischen Testelements (120), wenn mindestens eine vorbestimmte Dauer einer maximalen Wartezeit seit dem Zeitpunkt, der durch den Zeitstempel definiert ist, überschritten wurde;
- Bestimmen der mindestens einen Eigenschaft der Probe unter Verwendung mindestens des zweiten Bildes des jeweiligen optischen Testelements (120).

2. Analytisches Verfahren nach dem vorhergehenden Anspruch, wobei das Verfahren in Schritt iv. das Ableiten mindestens eines zweiten Elements von Identitätsinformationen auf dem mindestens einen optischen Testelement (120) von der eindeutigen Kennung (124) in dem zweiten Bild umfasst, wobei das Verifizieren, ob die eindeutigen Kennungen (124) in dem ersten Bild und in dem zweiten Bild identisch sind, das Verifizieren umfasst, ob das zweite Element von Identitätsinformationen mit mindestens einem der ersten Elemente von Identitätsinformationen in der Datenbank (128) identisch ist.

3. Analytisches Verfahren nach einem der vorhergehenden Ansprüche, wobei das analytische Verfahren das Verwenden einer Vielzahl von Testproben und einer Vielzahl von optischen Testelementen (120) umfasst, wobei jede Testprobe einem optischen Testelement (120) zugeordnet ist, wobei
- Schritt i. das Aufnehmen mindestens eines ersten Bildes für jedes der optischen Testelemente (120) umfasst, wodurch ein Satz von ersten Bildern erzeugt wird;
- Schritt iii. das Aufnehmen mindestens eines zweiten Bildes für jedes der optischen Testelemente (120) umfasst, wodurch ein Satz von zweiten Bildern erzeugt wird; und
- Schritt iv. für jedes der zweiten Bilder des Satzes von zweiten Bildern das Verifizieren umfasst, ob die eindeutige Kennung (124) in dem jeweiligen zweiten Bild auch in mindestens einem ersten Bild des Satzes von ersten Bildern vorhanden ist;
wobei das Verifizieren in Schritt iv. optional für jedes der optischen Testelemente (120) einzeln und unabhängig durchgeführt wird.

4. Analytisches Verfahren nach einem der vorhergehenden Ansprüche, wobei das Verfahren die Massenuntersuchung einer Vielzahl von Proben in einem Testzentrum umfasst.

5. Verfahren zum Betreiben der ersten mobilen Vorrichtung (112) mit den Merkmalen wie in Anspruch 1 definiert, wobei das Verfahren umfasst, dass die erste mobile Vorrichtung (112) mindestens die Schritte i. und ii. des Verfahrens nach einem der vorhergehenden Ansprüche, die sich auf ein analytisches Verfahren beziehen, durchführt, wobei das Verfahren ferner umfasst, dass der Prozessor (118) der ersten mobilen Vorrichtung (112) das mindestens eine erste Element von Identitätsinformationen auf dem mindestens einen optischen Testelement (120) von der eindeutigen Kennung (124) in dem ersten Bild ableitet, wobei die Datenbank (128) eine Cloud-basierte Datenbank ist, wobei die Cloud-basierte Datenbank eine Datenspeichervorrichtung aufweist, die unabhängig von der ersten mobilen Vorrichtung (112) und der zweiten mobilen Vorrichtung (114) ist, und über mindestens eine Datenverbindung zugänglich ist.

6. Verfahren nach dem vorhergehenden Anspruch, wobei Schritt ii. vor dem Speichern des Zeitstempels und des ersten Elements von Identitätsinformationen das Prüfen umfasst, ob das optische Testelement (120) bereits verwendet wurde, wobei Schritt ii. das Prüfen umfasst, ob das erste Element von Identitätsinformationen bereits in der Datenbank (128) gespeichert wurde, wobei, wenn das erste Element von Identitätsinformationen bereits in der Datenbank (128) gespeichert wurde, das jeweilige optische Testelement (120) als verwendet klassifiziert wird und das erste Bild abgelehnt wird, wobei, wenn das erste Element von Identitätsinformationen nicht in der Datenbank (128) gespeichert ist, das jeweilige optische Testelement (120) als nicht verwendet klassifiziert wird und das Verfahren mit dem Speichern des Zeitstempels und des jeweiligen ersten Elements von Identitätsinformationen fortfährt.

7. Verfahren zum Betreiben der zweiten mobilen Vorrichtung (114) mit den Merkmalen wie in Anspruch 1 definiert, wobei das Verfahren das Durchführen mindestens der Schritte iii. und iv. des Verfahrens nach einem der vorhergehenden Ansprüche, die sich auf ein analytisches Verfahren beziehen, umfasst, wobei das Verfahren ferner umfasst, dass der Prozessor (118) der zweiten mobilen Vorrichtung (114) in Schritt iv. mindestens ein zweites Element von Identitätsinformationen auf dem mindestens einen optischen Testelement (120) von der eindeutigen Kennung (124) in dem zweiten Bild ableitet, wobei das Verfahren ferner umfasst, dass der Prozessor (118) der zweiten mobilen Vorrichtung (114) in Schritt iv. unter Verwendung des Verfahrens nach dem vorhergehenden Anspruch verifiziert, ob das zweite Element von Identitätsinformationen mit mindestens einem der ersten Elemente von Identitätsinformationen in der Datenbank (128) identisch ist, in der eine Vielzahl von ersten Elementen von Identitätsinformationen gespeichert ist.

8. Computerprogramm, umfassend Anweisungen, die, wenn das Programm durch den Prozessor (118) der ersten mobilen Vorrichtung (112) mit den Merkmalen wie in Anspruch 1 definiert ausgeführt wird, den Prozessor (118) dazu veranlassen, die erste mobile Vorrichtung (112) zum Durchführen des Verfahrens nach Anspruch 5 zu steuern.

9. Computerlesbares Speichermedium, umfassend Anweisungen, die, wenn die Anweisungen durch den Prozessor (118) der ersten mobilen Vorrichtung (112) mit den Merkmalen wie in Anspruch 1 definiert ausgeführt werden, den Prozessor (118) dazu veranlassen, die erste mobile Vorrichtung (112) zum Durchführen des Verfahrens nach Anspruch 5 zu steuern.

10. Computerprogramm, umfassend Anweisungen, die, wenn das Programm durch den Prozessor (118) der zweiten mobilen Vorrichtung (114) mit den Merkmalen wie in Anspruch 1 definiert ausgeführt wird, den Prozessor (118) dazu veranlassen, die zweite mobile Vorrichtung (114) zum Durchführen des Verfahrens nach Anspruch 7 zu steuern.

11. Computerlesbares Speichermedium, umfassend Anweisungen, die, wenn die Anweisungen durch den Prozessor (118) der zweiten mobilen Vorrichtung (114) mit den Merkmalen wie in Anspruch 1 definiert ausgeführt werden, den Prozessor (118) dazu veranlassen, die zweite mobile Vorrichtung (114) zum Durchführen des Verfahrens nach Anspruch 7 zu steuern.

12. Erste mobile Vorrichtung (112), umfassend mindestens eine Kamera (116) und mindestens einen Prozessor (118), wobei die erste mobile Vorrichtung (112) zum Durchführen des Verfahrens nach Anspruch 5 konfiguriert ist.

13. Zweite mobile Vorrichtung (114), umfassend mindestens eine Kamera (116) und mindestens einen Prozessor (118), wobei die zweite mobile Vorrichtung (114) zum Durchführen des Verfahrens nach Anspruch 7 konfiguriert ist.

14. Analytisches System (110), umfassend mindestens eine erste mobile Vorrichtung (112) und mindestens eine zweite mobile Vorrichtung (114), wobei jede der ersten (112) und zweiten mobilen Vorrichtung (114) mindestens eine Kamera (116) und mindestens einen Prozessor (118) umfasst, wobei das analytische System (110) ferner mindestens ein optisches Testelement (120) umfasst, das mindestens einen Reagenztestbereich (122) und mindestens eine eindeutige Kennung (124) umfasst, die dem optischen Testelement (120) zugeordnet ist, wobei das analytische System (110) ferner mindestens eine Datenbank (128) umfasst, wobei das analytische System (110) zum Durchführen des analytischen Verfahrens nach einem der vorhergehenden Ansprüche, die sich auf ein analytisches Verfahren beziehen, konfiguriert ist.

15. Computerprogramm, umfassend Anweisungen, die, wenn das Programm durch das analytische System (110) nach Anspruch 14 ausgeführt wird, das analytische System (110) dazu veranlassen, das analytische Verfahren nach einem der vorhergehenden Ansprüche, die sich auf ein analytisches Verfahren beziehen, durchzuführen.

16. Computerlesbares Speichermedium, umfassend Anweisungen, die, wenn die Anweisungen durch das analytische System (110) nach Anspruch 14 ausgeführt werden, das analytische System (110) dazu veranlassen, das analytische Verfahren nach einem der vorhergehenden Ansprüche, die sich auf ein analytisches Verfahren beziehen, durchzuführen.

## Revendications

1. Procédé analytique pour déterminer au moins une propriété d'au moins un échantillon d'un fluide corporel, le procédé utilisant au moins un premier dispositif mobile (112) et au moins un deuxième dispositif mobile (114), chacun desdits dispositifs mobiles (112, 114) comprenant au moins une caméra (116) et au moins un processeur (118), le procédé utilisant en outre au moins un élément de test optique (120) comprenant au moins une région de test de réactif (122) et au moins un identifiant unique (124) associé à l'élément de test optique (120), le procédé comprenant en outre l'utilisation d'au moins une base de données (128), le procédé comprenant :
i. la capture d'au moins une première image en utilisant la caméra (116) du premier dispositif mobile (112), avant d'appliquer l'échantillon du fluide corporel à la région de test de réactif (122) de l'élément de test optique (120), ladite première image comprenant au moins une partie de l'identifiant unique (124) ;
ii. le stockage d'au moins un horodatage et d'au moins un premier élément d'information d'identité sur l'au moins un élément de test optique (120) dans la base de données (128), l'au moins un premier élément d'information d'identité étant dérivé de l'identifiant unique (124) dans la première image ;
iii. la capture d'au moins une deuxième image en utilisant la caméra (116) du deuxième dispositif mobile (114), ladite deuxième image comprenant au moins une partie de l'au moins un identifiant unique (124) et au moins une partie de la région de test de réactif (122) de l'élément de test optique (120) ayant l'échantillon du fluide corporel appliqué sur celle-ci ;
iv. la vérification, en utilisant la base de données (128), si les identifiants uniques (124) dans la première image et dans la deuxième image sont identiques et la vérification si au moins une période prédéterminée de temps d'attente minimal s'est écoulée depuis un point dans le temps défini par l'horodatage, dans lequel le procédé comprend en outre, en se basant sur le résultat des vérifications, la réalisation d'une action choisie dans le groupe d'actions comprenant :
- l'abandon de l'évaluation ultérieure de l'élément de test optique (120) respectif si les identifiants uniques (124) dans les première et deuxième images ne sont pas identiques ;
- l'abandon de l'évaluation ultérieure de l'élément de test optique (120) respectif si la période prédéterminée de temps d'attente minimal depuis le point dans le temps défini par l'horodatage ne s'est pas encore écoulée ;
- l'abandon de l'évaluation ultérieure de l'élément de test optique (120) respectif si au moins une période prédéterminée de temps d'attente maximal depuis le point dans le temps défini par l'horodatage a été dépassée ;
- la détermination de l'au moins une propriété de l'échantillon en utilisant au moins la deuxième image de l'élément de test optique (120) respectif.

2. Procédé analytique selon la revendication précédente, dans lequel le procédé, à l'étape iv., comprend la dérivation d'au moins un deuxième élément d'information d'identité sur l'au moins un élément de test optique (120) à partir de l'identifiant unique (124) dans la deuxième image, dans lequel la vérification si les identifiants uniques (124) dans la première image et dans la deuxième image sont identiques comprend la vérification si le deuxième élément d'information d'identité est identique à au moins un parmi les premiers éléments d'information d'identité dans la base de données (128).

3. Procédé analytique selon l'une quelconque des revendications précédentes, dans lequel le procédé analytique comprend l'utilisation d'une pluralité d'échantillons de test et d'une pluralité d'éléments de test optique (120), dans lequel chaque échantillon de test est affecté à un élément de test optique (120), dans lequel
- l'étape i. comprend la capture d'au moins une première image pour chacun des éléments de test optique (120), générant ainsi un ensemble de premières images ;
- l'étape iii. comprend la capture d'au moins une deuxième image pour chacun des éléments de test optique (120), générant ainsi un ensemble de deuxièmes images ; et
- l'étape iv. comprend la vérification, pour chacune des deuxièmes images de l'ensemble de deuxièmes images, si l'identifiant unique (124) dans la deuxième image respective est également présent dans au moins une première image de l'ensemble de premières images ;
dans lequel, éventuellement, la vérification à l'étape iv. est réalisée pour chacun des éléments de test optique (120) de manière individuelle et indépendante.

4. Procédé analytique selon l'une quelconque des revendications précédentes, dans lequel le procédé comprend des tests de masse d'une pluralité d'échantillons dans un centre de test.

5. Procédé de fonctionnement du premier dispositif mobile (112) avec les caractéristiques telles que définies dans la revendication 1, le procédé comprenant la réalisation par le premier dispositif mobile (112) d'au moins les étapes i. et ii. du procédé selon l'une quelconque des revendications précédentes se référant à un procédé analytique, le procédé comprenant en outre la dérivation par le processeur (118) du premier dispositif mobile (112) de l'au moins un premier élément d'information d'identité sur l'au moins un élément de test optique (120) à partir de l'identifiant unique (124) dans la première image, dans lequel la base de données (128) est une base de données basée sur le cloud, dans lequel la base de données basée sur le cloud a un dispositif de stockage de données indépendant du premier dispositif mobile (112) et du deuxième dispositif mobile (114) et est accessible par l'intermédiaire d'au moins une connexion de données.

6. Procédé selon la revendication précédente, dans lequel l'étape ii. comprend, avant le stockage de l'horodatage et du premier élément d'information d'identité, la vérification si l'élément de test optique (120) a déjà été utilisé, dans lequel l'étape ii. comprend la vérification si le premier élément d'information d'identité a déjà été stocké dans la base de données (128), dans lequel, si le premier élément d'information d'identité est déjà stocké dans la base de données (128), l'élément de test optique (120) respectif est classé comme étant utilisé et la première image est refusée, dans lequel, si le premier élément d'information d'identité n'est pas stocké dans la base de données (128), l'élément de test optique (120) respectif est classé comme étant inutilisé et le procédé se poursuit en stockant l'horodatage et le premier élément d'information d'identité respectif.

7. Procédé de fonctionnement du deuxième dispositif mobile (114) avec les caractéristiques telles que définies dans la revendication 1, le procédé comprenant la réalisation d'au moins les étapes iii. et iv. du procédé selon l'une quelconque des revendications précédentes se référant à un procédé analytique, le procédé comprenant en outre la dérivation par le processeur (118) du deuxième dispositif mobile (114), à l'étape iv., d'au moins un deuxième élément d'information d'identité sur l'au moins un élément de test optique (120) à partir de l'identifiant unique (124) dans la deuxième image, le procédé comprenant en outre la vérification par le processeur (118) du deuxième dispositif mobile (114), à l'étape iv., si le deuxième élément d'information d'identité est identique à au moins l'un des premiers éléments d'information d'identité dans la base de données (128) ayant stockés dans celle-ci une pluralité de premiers éléments d'information d'identité en utilisant le procédé selon la revendication précédente.

8. Programme informatique comprenant des instructions qui, lorsque le programme est exécuté par le processeur (118) du premier dispositif mobile (112) avec les caractéristiques telles que définies dans la revendication 1, amènent le processeur (118) à commander le premier dispositif mobile (112) afin de réaliser le procédé selon la revendication 5.

9. Support de stockage lisible par ordinateur comprenant des instructions qui, lorsque les instructions sont exécutées par le processeur (118) du premier dispositif mobile (112) avec les caractéristiques telles que définies dans la revendication 1, amènent le processeur (118) à commander le premier dispositif mobile (112) afin de réaliser le procédé selon la revendication 5.

10. Programme informatique comprenant des instructions qui, lorsque le programme est exécuté par le processeur (118) du deuxième dispositif mobile (114) avec les caractéristiques telles que définies dans la revendication 1, amènent le processeur (118) à commander le deuxième dispositif mobile (114) afin de réaliser le procédé selon la revendication 7.

11. Support de stockage lisible par ordinateur comprenant des instructions qui, lorsque les instructions sont exécutées par le processeur (118) du deuxième dispositif mobile (114) avec les caractéristiques telles que définies dans la revendication 1, amènent le processeur (118) à commander le deuxième dispositif mobile (114) afin de réaliser le procédé selon la revendication 7.

12. Premier dispositif mobile (112) comprenant au moins une caméra (116) et au moins un processeur (118), dans lequel le premier dispositif mobile (112) est configuré pour réaliser le procédé selon la revendication 5.

13. Deuxième dispositif mobile (114) comprenant au moins une caméra (116) et au moins un processeur (118), dans lequel le deuxième dispositif mobile (114) est configuré pour réaliser le procédé selon la revendication 7.

14. Système analytique (110) comprenant au moins un premier dispositif mobile (112) et au moins un deuxième dispositif mobile (114), dans lequel chacun des premier (112) et deuxième dispositifs mobiles (114) comprend au moins une caméra (116) et au moins un processeur (118), le système analytique (110) comprenant en outre au moins un élément de test optique (120) comprenant au moins une région de test de réactif (122) et au moins un identifiant unique (124) associé à l'élément de test optique (120), le système analytique (110) comprenant en outre au moins une base de données (128), dans lequel le système analytique (110) est configuré pour réaliser le procédé analytique selon l'une quelconque des revendications précédentes se référant à un procédé analytique.

15. Programme informatique comprenant des instructions qui, lorsque le programme est exécuté par le système analytique (110) selon la revendication 14, amènent le système analytique (110) à réaliser le procédé analytique selon l'une quelconque des revendications précédentes se référant à un procédé analytique.

16. Support de stockage lisible par ordinateur comprenant des instructions qui, lorsque les instructions sont exécutées par le système analytique (110) selon la revendication 14, amènent le système analytique (110) à réaliser le procédé analytique selon l'une quelconque des revendications précédentes se référant à un procédé analytique.
